(19) Europäisches Patentamt
European Patent Office
Office européen des brevets

(11) Veröffentlichungsnummer : **0 131 258 B1**

(12) # EUROPÄISCHE PATENTSCHRIFT

(45) Veröffentlichungstag der Patentschrift : **30.08.89**

(21) Anmeldenummer : **84107833.0**

(22) Anmeldetag : **05.07.84**

(51) Int. Cl.⁴ : **C 07 D251/16**, C 07 D251/46, C 07 D239/42, C 07 D239/47, C 07 D239/52, C 07 D513/04, A 01 N 43/54, A 01 N 47/36 // (C07D513/04, 285:00, 251:00),(C07D513/04, 285:00, 239:00)

(54) **Neue N-Alkoxy- N-Alkylsulfonylaminosulfonylharnstoffe, und neue (Pyrimido) Triazino-thiatriazinoxide als Vorprodukte.**

(30) Priorität : **09.07.83 DE 3324802**

(43) Veröffentlichungstag der Anmeldung : **16.01.85 Patentblatt 85/03**

(45) Bekanntmachung des Hinweises auf die Patenterteilung : **30.08.89 Patentblatt 89/35**

(84) Benannte Vertragsstaaten : **AT BE CH DE FR GB IT LI NL SE**

(56) Entgegenhaltungen :
**EP-A- 0 072 347**
**DE-A- 2 202 557**
**DE-A- 2 257 240**
**DE-A- 3 243 533**
**DE-A- 3 300 569**
**GB-A- 2 015 503**
**CHEMICAL ABSTRACTS, Band 91, Nr. 11, 10. September 1979, Seite 778, Zusammenfassungsnr. 91605u, Columbus, Ohio, US; S. KARADY et al.: "Heterocycles from chlorosulfonyl isocyanate. I. Reaction with 2-aminoazines"**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Willms, Lothar, Dr.**
**Grüner Weg 4**
**D-5463 Unkel (DE)**
Erfinder : **Mildenberger, Hilmar, Dr.**
**Fasanenstrasse 24**
**D-6233 Kelkheim (Taunus) (DE)**
Erfinder : **Bauer, Klaus, Dr.**
**Kolpingstrasse 7**
**D-6054 Rodgau (DE)**
Erfinder : **Bürstell, Helmut, Dr.**
**Am Hohlacker 65**
**D-6000 Frankfurt am Main 50 (DE)**
Erfinder : **Bieringer, Hermann, Dr.**
**Eichenweg 26**
**D-6239 Eppstein/Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 131 258 B1**

Jouve, 18, rue St-Denis, 75001 Paris, France

**Beschreibung**

Es ist bereits bekannt, daß heterocyclisch substituierte Phenylsulfonylharnstoffe herbizide oder pflanzenwuchsregulierende Eigenschaften aufweisen s. z. B. NL-A 121 788, DE-A 27 15 786, EP-A 1485, DE-A 3243 533. In DE-A 3 243 533 werden solche Harnstoffe beschrieben, die einen substituierten Amino-Rest an der Sulfonylgruppe tragen. Diese weisen jedoch Nachteile bei der Anwendung auf wie beispielsweise eine hohe Persistenz oder unzureichende Selektivität.

Es wurde nun gefunden, daß heterocyclisch substituierte N-Alkoxy- und N-Alkylsulfonylaminosulfonylharnstoffe, welche als heterocyclische Komponente einen Pyrimidin- oder Triazinring enthalten, als Herbizide und Pflanzenwachstumsregulatoren besonders geeignet sind.

Gegenstand der vorliegenden Erfindung sind demnach die neuen Verbindungen der allgemeinen Formel (I)

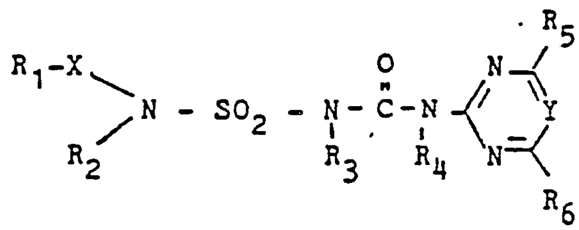

(I)

worin

X O oder $—SO_2—$,

Y CH oder Stickstoff,

$R_1$ einen $(C_1\text{-}C_6)$-Alkyl, $(C_2\text{-}C_6)$-Alkenyl oder einen $(C_2\text{-}C_6)$-Alkinylrest bedeutet, die gegebenenfalls durch Halogen, $(C_1\text{-}C_4)$-Alkoxy oder $(C_1\text{-}C_4)$-Alkoxycarbonyl substituiert sind,

$R_2$ Wasserstoff, $(C_1\text{-}C_6)$-Alkyl, $(C_2\text{-}C_6)$-Alkenyl, $(C_2\text{-}C_6)$-Alkinyl oder Cyclohexyl,

$R_3$, $R_4$ unabhängig voneinander Wasserstoff oder $(C_1\text{-}C_4)$-Alkyl,

$R_5$, $R_6$ unabhängig voneinander Wasserstoff, $(C_1\text{-}C_4)$-Alkyl oder $(C_1\text{-}C_4)$-Alkoxy, die gegebenenfalls ein oder mehrfach durch Halogen, $(C_1\text{-}C_4)$-Alkoxy oder $(C_1\text{-}C_4)$-Alkylthio substituiert sein können, Halogen, $(C_1\text{-}C_4)$-Alkylthio, $(C_1\text{-}C_4)$-Alkylamino oder $(C_1\text{-}C_4)$-Dialkylamino bedeuten,

sowie, falls $R_2$, $R_3$ Wasserstoff bedeuten, deren physiologisch verträgliche Salze mit Basen.

« Halogen » bedeutet bevorzugt Fluor, Chlor oder Brom.

Unter den Verbindungen der Formel I sind diejenigen bevorzugt, in den $R_1$, $R_2$ unabhängig voneinander $(C_1\text{-}C_3)$-Alkyl oder Alkenyl mit bis zu 3 C-Atomen, $R_3$ und $R_4$ Wasserstoff, $R_5$, $R_6$ unabhängig voneinander $(C_1\text{-}C_2)$-Alkyl oder $(C_1\text{-}C_2)$-Alkoxy, $X=SO_2$ und $Y=CH$ bedeuten.

Die Erfindung betrifft ebenfalls neue (Pyrimido) Triazino-thiatriazinoxide der Formel (II), die wie unten ausgeführt, als Zwischenprodukte zur Herstellung der erfindungsgemäßen Verbindungen der Formel (I) fungieren können.

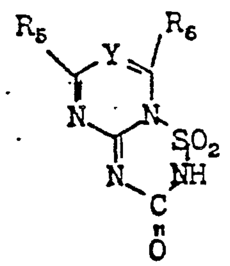

(II)

Die neuen Verbindungen der allgemeinen Formel I lassen sich aus an sich bekannten oder aus nach bekannten Verfahren hergestellten Ausgangsmaterialien synthetisieren.

Die Herstellungsverfahren sind dadurch gekennzeichnet, daß man

a) Verbindungen der Formel (III), worin

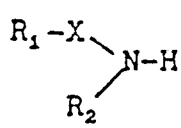

(III)

$R_1$, $R_2$ und X die obengenannten Bedeutungen besitzen, mit Verbindungen der Formel (IV),

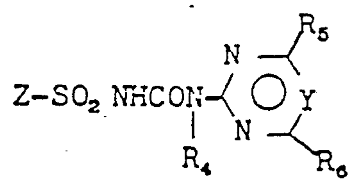

(IV)

worin Z=Fluor, Chlor, ($C_1$-$C_4$)-Alkoxy- oder Phenoxy, die gegebenenfalls halogeniert sind, insbesondere Chlor bedeutet und $R_4$, $R_5$, $R_6$ und Y den Definitionen für die Verbindungen der Formel (I) entsprechen oder

b) Verbindungen der Formel (II)

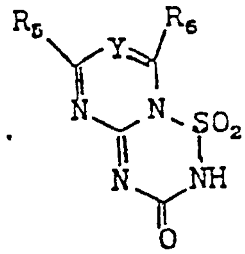

(II)

worin Y, $R_5$ und $R_6$ den Definitionen für Verbindungen der Formel (I) entsprechen, mit Verbindungen der obengenannten Formel (III), oder

c) im Falle X = $SO_2$ Verbindungen der Formel (V),

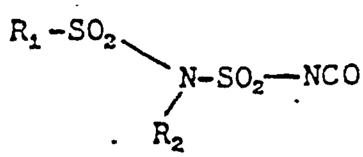

(V)

worin $R_1$ und $R_2$ den für Verbindungen der Formel (I) gegebenen Definitionen entsprechen, mit Verbindungen der Formel (VI), worin $R_4$, $R_5$, $R_6$ und Y die obengenannten Bedeutungen besitzen,

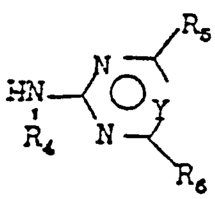

(VI)

umsetzt, und gegebenenfalls die gemäß a) bis c) erhaltenen Verbindungen der Formel (I), worin $R_2$, $R_3$ unabhängig voneinander Wasserstoff bedeuten, in $R_2$ oder $R_3$-Position alkyliert.

Zu a) Die Umsetzung der Verbindungen (III) und (IV) erfolgt vorzugsweise in inerten aprotischen Lösungsmitteln wie aliphatischen oder aromatischen Kohlenwasserstoffen insbesondere n-Hexan, Toluol oder Xylol, Acetonitril, Dichlormethan, Chloroform, Nitromethan, Tetrahydrofuran, oder Dioxan bei Temperaturen zwischen — 78 °C und der Siedetemperatur des Lösungsmittels, vorzugsweise in Gegenwart eines Säureakzeptors wie eines tertiären organischen Amins, insbesondere Pyridin oder Triethylamin oder einer anorganischen Base wie einem Alkalicarbonat. Gegebenenfalls kann im Falle X=Sauerstoff die Verbindung (III) im Überschuß eingesetzt werden, um so gleichzeitig als Hilfsbase zu fungieren.

Zu b) Bei der Umsetzung der Verbindungen (II) und (III) verwendet man vorzugsweise die gleichen Lösungsmittel wie bei a) und arbeitet bei Temperaturen zwischen — 78 °C und der Siedetemperatur des Lösungsmittels. Gegebenenfalls wird die Reaktion unter Zusatz von einem oder mehreren Äquivalenten basischer Verbindungen, wie tertiären organischen Aminen, insbesondere Triethylamin, Ethyldiisopropylamin, Pyridin oder an organischen Basen wie Kaliumcarbonat durchgeführt.

Zu c) Auch die Acylierung von Aminoheterocyclen der Formel VI mit Sulfonylisocyanaten der Formel V wird vorzugsweise in den gleichen unter a) genannten Lösungsmitteln bei Temperaturen zwischen — 20 °C und der Siedetemperatur des Lösungsmittels durchgeführt.

Zur nachträglichen Alkylierung der Verbindungen der Formel I in $R_2$- bzw. $R_3$-Position arbeitet man vorzugsweise in inerten Lösungsmitteln wie z. B. Dioxan oder Dimethylformamid unter Zusatz einer

anorganischen Base wie Natriumhydrid oder Kaliumcarbonat, bei temperaturen von 0 °C bis zur Siedetemperatur des Lösungsmittels. Als Alkylierungsmittel werden übliche Mittel wie Dimethylsulfat, Methyljodid oder Ethylbromid eingesetzt.

Verbindungen der Formel I, in denen $R_2$ oder $R_3$ Wasserstoff bedeutet, können Salze bilden, bei denen H durch ein geeignetes Kation ersetzt ist. Diese Salze sind im allgemeinen Metall-, insbesondere Alkali- oder Erdalkalisalze, gegebenenfalls alkylierte Ammonium- oder organische Aminsalze und werden vorzugsweise in inerten Lösungsmitteln wie z. B. Wasser, Methanol oder Aceton bei Temperaturen von 20-100 °C hergestellt. Geeignete Basen zur Herstellung der erfindungsgemäßen Salze sind z. B. Alkalicarbonate, Ammoniak oder Ethanolamin.

Die für die Herstellung der erfindungsgemäßen Verbindungen der allgemeinen Formel (I) benötigten Ausgangsstoffe der Formel (II) sind neu und lassen sich durch Anlagerung von Chlor- oder Fluorsulfonylisocyanat an Aminoheterocyclen der obengenannten Formel (VI) in Gegenwart von Basen wie tertiären organischen Aminen, insbesondere Triethylamin oder Ethyldiisopropylamin, herstellen. Die Verbindungen der Formel II und ihr Herstellungsverfahren sind daher ebenfalls Gegenstand der Erfindung. Dieses Verfahren wird vorzugsweise in inerten organischen Lösungsmitteln wie Dichlormethan, Acetonitril, Tetrahydrofuran bei Temperaturen zwischen — 78 °C und + 40 °C durchgeführt. Für $R_5$, $R_6$=($C_1$-$C_4$)Alkylamino oder ($C_1$-$C_4$) Dialkylamino ist diese Herstellungsmethode der Verbindungen der Formel II wegen geringer Ausbeuten weniger geeignet. Bei dem genannten Herstellungsverfahren fallen die Verbindungen der Formel II im Falle $R_5$=$R_6$ als Isomerengemische an. Es handelt sich hierbei um Gemische zweier Stellungsisomeren, wobei bei beiden Isomeren die Bedeutungen von $R_5$ und $R_6$ vertauscht sind, da der Ringschluß der Sulfonylgruppe des Halogensulfonylisocyanats an beiden der Aminogruppe benachbarten Stickstoffatome von Formel VI erfolgen kann. Die Auftrennung dieser Regioisomerengemische kann nach üblichen Methoden wie Umkristallisation, Chromatographie usw. erfolgen.

Die Ausgangsstoffe der Formel III sind bekannt oder lassen sich nach im Prinzip bekannten Verfahren herstellen. Die entsprechenden Hydroxylamine der Formel (III) (X=O) werden z. B. durch alkalische Hydrolyse von Alkylalkoxycarbaminsäureestern s. Angew. 75, 851 (1963) hergestellt.

Die aliphatischen Sulfonamide der Formel (III) ($X=SO_2$) lassen sich z. B. aus Alkylsulfochloriden durch Umsetzung mit primären aliphatischen Aminen herstellen.

Die heterocyclischen Sulfonylharnstoffe der Formel (IV) sind bekannt oder lassen sich nach bekannten Methoden herstellen für Z=Chlor s. EP-A-39 239, für Z=Alkoxy s. EP-A-61 661, für Z=Phenoxy s. GB-A-2015 503.

Die Sulfonylisocyanate der Formel (V) werden durch Umsetzung von Chlorsulfonylisocyanat mit sekundären aliphatischen Sulfonamiden hergestellt (DE-A-2 257 240).

Die Ausgangsstoffe der Formel IV sind bekannt oder lassen sich nach im Prinzip bekannten Verfahren herstellen, z. B. durch Zyklisierung entsprechender Guanidinderivate mit entsprechend substituierten 1,3-Diketonen, vergleiche z. B. « The Chemistry of Heterocyclic compounds », Vol. XVI (1962) und Supplement I (1970) oder durch Derivatisierung von Cyanurchlorid, vergleiche z. B. « The Chemistry of Heterocyclic Compounds », L. Rapoport : « s-Triazines and Derivatives » (1959).

Die erfindungsgemäßen heterozyklischen Sulfonylharnstoffderivate weisen eine ausgezeichnete herbizide Wirkung und in wichtigen Großkulturen eine sehr gute Selektivität auf. Sie eignen sich daher zur selektiven Bekämpfung zweikeimblättriger und grasartiger annueller und perennierender Unkräuter, insbesondere in landwirtschaftlich bedeutenden Kulturen wie z. B. Weizen, Gerste, Roggen, Reis, Mais, Zuckerrübe und Soja. Dabei ist es gleichgültig, ob die Substanzen in Vorsaat-, Vorauflauf- oder Nachauflaufspritzung ausgebracht werden. Werden die erfindungsgemäßen Verbindungen vor dem Keimen der Unkrautpflanzen im Vorsaat- oder Vorauflaufverfahren auf die Erdoberfläche appliziert, so wird das Auflaufen der Keimlinge nicht verhindert. Die Unkräuter wachsen bis zum Keimblattstadium heran, stellen jedoch dann ihr Wachstum ein und sterben schließlich nach 3-5 Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. durch Auslösen von Desikkation und Wuchsstauchung eingesetzt werden. Des weiteren eignen sie sich auch zur generellen Steuerung und Hemmung von unerwünschten vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Besonders hervorzuheben ist die wachstumsregulatorische Wirksamkeit der Verbindungen als Wuchshemmer in Getreide, Mais, Soja, Baumwolle, Rasen sowie ihre Fähigkeiten, den Gehalt an erwünschten Inhaltsstoffen wie Kohlehydraten und Protein bei Nutzpflanzen zu erhöhen. Schließlich zeigen die Verbindungen eine sehr gute Verbesserung der Fruchtabszission insbesondere bei Zitrusfrüchten oder Reduktion der Haltekraft.

Gegenstand der Erfindung sind daher auch herbizide und wachstumsregulierende Mittel, die gekennzeichnet sind durch einen Gehalt an einer Verbindung der Formel I in Kombination mit üblichen Formulierungshilfsmitteln und Inertstoffen sowie deren Verwendung im landwirtschaftlichen Bereich.

Die erfindungsgemäßen Mittel enthalten die Wirkstoffe der Formel I im allgemeinen zu 2-95 Gew.-%. Sie können als Spritzpulver, emulgierbare Konzentrate, versprühbare Lösungen, Stäubemittel oder Granulate in den üblichen Zubereitungen angewendet werden.

Die Spritzpulver sind in Wasser gleichmäßig dispergierbare Präparate, die neben dem Wirkstoff außer einem Verdünnungsmittel oder Inertstoff noch Netzmittel z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden :

Alkylarylsulfonsaure Kalziumsalze wie Ca-dodecyl-benzosulfonat oder nichtionische Emulgatoren wie fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxethylensorbitfett-säureester oder Polyoxethylen-sorbitester.

Stäubemittel erhält man durch Vermahlen des Wirkstoffes mit fein verteilten, festen Stoffen, z. B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise — gewünschtenfalls in Mischung mit Düngemitteln — hergestellt werden.

Bei herbiziden Mitteln können die Konzentrationen der Wirkstoffe in den handelsüblichen Formulierungen verschieden sein.

In Spritzpulvern variiert die Wirkstoffkonzentration z. B. zwischen etwa 10 % und 80 %, der Rest besteht aus den oben angegebenen Formulierungszusätzen. Bei emulgierbaren Konzentraten kann die Wirkstoffkonzentration gleichfalls etwa 10 % bis 80 % betragen. Staubförmige Formulierungen enthalten etwa 2-20 %. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Zur Anwendung als Herbizide werden die handelsüblichen Konzentrate gegebenenfalls in üblicher Weise verdünnt, z. B. bei Spritzpulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit, u. a. variiert die erforderliche Aufwandmenge. Sie beträgt im allgemeinen zwischen 0.01 und 10 kg/ha, vorzugsweise etwa 0.1 bis 5.0 kg/ha Wirkstoff.

Für einige Anwendungsgebiete kann es zweckmäßig sein, die neuen Herbizide mit einem oder mehreren Herbiziden gemeinsam anzuwenden, z. B. als Tankmischung oder in Form einer Fertigformulierung, um weitere vorteilhafte Wirkungen zu erzielen.

Die erfindungsgemäßen Wirkstoffe können mit anderen Herbiziden, Insektiziden und Fungiziden kombiniert werden.

Zur Anwendung als Wachstumsregularoten eignen sich Konzentrationen zwischen 0.01 und 1.25 kg/ha. Bevorzugt verwendet man wäßrige Dispersionen von Spritzpulvern oder Verdünnungen von emulgierbaren Konzentraten. Die Anwendung erfolgt im Nachauflauf. Bevorzugte Kulturen sind Mais und Tabak.

## Herstellungsbeispiele

### Beispiel 1

2,3-Dihydro-6,8-dimethyl-3-oxo-pyrimido-[1,2-b] [1,2,4,6] thiatriazin-1,1-dioxid

17,5 ml (0,2 Mol) Chlorsulfonylisocyanat (CSI) werden in 300 ml wasserfreiem $CH_3CN$ gelöst und bei — 40 °C unter Stickstoff in ca. 5 min. mit 24,6 g (0,2 Mol) 2-Amino-4,6-dimethylpyrimidin versetzt. Nach Entfernen des Kältebades läßt man das Reaktionsgemisch in ca. 1,5 h auf 10 °C kommen (ab — 5 °C nimmt der Kolbeninhalt eine tiefrote Färbung an).

Nach 18 h Nachrühren bei RT wird der Niederschlag abgesaugt, mit $CH_3CN$, anschließend mit n-Hexan gewaschen und bei 60 °C im Hochvakuum getrocknet.

Man erhält 21,5 g (42,2 % d. Th.) 2,3-Dihydro-6,8-dimethyl-3-oxo-pyrimido-[1,2-b] [1,2,4,6] thiatriazin-1,1-dioxid. Fp. 240 °C (Z.)

In analoger Weise erhält man die in nachfolgender Tabelle angegebenen Verbindungen der

Formel (II).

| Beispiel-Nr. | $R_5$ (bzw. $R_6$ ) | $R_6$ (bzw. $R_5$) | Y | physikal. Daten |
|---|---|---|---|---|
| 2 | $CH_3$ | $OCH_3$ | CH | Smp. 294 - 297°C |
| 3 | $CH_3$ | H | CH | |
| 4 | $CH_3$ | Cl | CH | Smp. 271°C (Z.) |
| 5 | $OCH_3$ | $OCH_3$ | CH | Smp. 272 - 6°C |
| 6 | $CH_3$ | $OC_2H_5$ | CH | |
| 7 | $C_2H_5$ | $OCH_3$ | CH | |
| 8 | $CH_2SCH_3$ | $OCH_3$ | CH | |
| 9 | $CH_2OCH_3$ | $OCH_3$ | CH | Smp. 186°C (Z.) |
| 10 | $CH_3$ | $OCH_3$ | N | |
| 11 | $OCH_3$ | $OCH_3$ | N | |
| 12 | $CH_2Cl$ | $OCH_3$ | N | |
| 13 | $CH_2OCH_3$ | $OCH_3$ | N | |
| 14 | $OC_2H_5$ | $CH_3$ | N | |
| 15 | $C_2H_5$ | $OCH_3$ | N | |
| 16 | $CH_2SCH_3$ | $OCH_3$ | N | |

Die Verbindungen der Beispiele 2-16 liegen als Isomerengemische vor.

## Beispiel 17

1-[(N-Methoxy-N-methyl-amino)-sulfonyl]-3-(4-methoxy-6-methyl-2-pyrimidinyl)-harnstoff

14,8 g (0,105 Mol) CSI werden bei —70 °C unter Stickstoff in 150 ml Dichlormethan gelöst und protionsweise mit 13.9 g (0,1 Mol) 2-Amino-4-methoxy-6-methylpyrimidin versetzt.

Man läßt das Reaktionsgemisch innerhalb einer Stunde auf 0 °C kommen, kühlt auf —70 °C ab, fügt 9,35 g (0,1 Mol) O,N-Dimethylhydroxylamin-hydrochlorid hinzu und versetzt innerhalb einer weiteren Stunde mit 20,2 g (0,2 Mol) Triethylamin- gelöst in 100 ml Dichlormethan. Nach 2 Stunden Rühren bei —70 °C wird 18 Stunden bei Raumtemperatur nachgerührt, mit 0,5 n Natriumacetatlösung, anschließend mit gesättigter Kochsalzlösung gewaschen und die Dichlormethanphase über Natriumsulfat getrocknet. Nach Zutropfen von n-Hexan werden 19,8 g (55,3 % d. Th.) an 1-[(N-Methoxy-N-methyl-amino)-sulfonyl]-3-(4-methoxy-6-methyl-2-pyrimidinyl)-harnstoff vom Schmelzpunkt 154 °C erhalten.

## Beispiel 18

1-[(N-Methoxy-N-methyl-amino)-sulfonyl]-3-(4,6-dimethyl-2-pyrimidinyl)-harnstoff

5,7 g (0,025 Mol) 2,3-Dihydro-6,8-dimethyl-3-oxopyrimido-[1,2-b] [1,2,4,6]-thiatriazin-1,1-dioxid (vergleiche Beispiel 1) werden in 100 ml Dichlormethan suspendiert und bei —40 °C mit 3,05 g (0,05 Mol) O,N-Dimethylhydroxylamin — gelöst in 50 ml Dichlormethan — innerhalb von einer Stunde versetzt. Man rührt 18 Stunden bei Raumtemperatur nach, dampft bis zur Trockene ein, nimmt an Wasser auf und

säuert mit 0,5 n HCl auf pH 4-5 an. Nach Extraktion mit Dichlormethan wird wie unter Beispiel 17 aufgearbeitet. Man erhält 4,9 g (67,8 % d. Th.) an 1-[(N-Methoxy-N-methyl-amino)-sulfonyl]-3-(4,6-dimethyl-2-pyrimidinyl)-harnstoff vom Smp. 152 °C.

## Beispiel 19

1-[(N-Methylsulfonyl-N-methyl-amino)-sulfonyl]-3-(4-methoxy-6-methyl-2-pyrimidinyl)-harnstoff

13.9 g (0,1 Mol) 2-Amino-4-methoxy-6-methylpyrimidin werden in 150 ml Dichlormethan suspendiert und bei 0 °C in einer Stunde mit 21,4 g (0,1 Mol) N-Methylsulfonyl-N-methyl-amino-sulfonylisocyanat in 50 ml Dichlormethan versetzt. Nach 15 Stunden Nachrühren bei Raumtemperatur wird das Produkt mit n-Hexan ausgefällt. Man erhält 20,4 g (58 % d. Th.) an 1-[(N-Methylsulfonyl-N-methyl-amino)-sulfonyl]-3-(4-methoxy-6-methyl-2-pyrimidinyl)-harnstoff vom SMP 118-120 °C.

Analog gemäß einem der Beispiele 17 bis 19 können die in nachfolgender Tabelle 2 aufgeführten Verbindungen der Formel I synthetisiert werden.

(Siehe Tabelle 2 Seite 8 ff.)

Herbizide Wirkung

Die vorliegenden erfindungsgemäßen Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende Wurzelunkräuter werden durch die Wirkstoffe gut erfaßt. Dabei ist es gleichgültig, ob die Substanzen in Vorsaat-, Vorauflauf- oder Nachauflaufspritzung ausgebracht werden.

Werden die erfindungsgemäßen Verbindungen vor dem Keimen der Unkrautpflanzen im Vorsaat- oder Vorauflaufverfahren auf die Erdoberfläche appliziert, so wird das Auflaufen der Keimlinge nicht verhindert. Die Unkräuter wachsen bis zum Keimblattstadium heran, stellen jedoch dann ihr Wachstum ein und sterben schließlich nach 3-5 Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja nur unwesentlich oder gar nicht geschädigt. Gegenüber dem Stand der Technik besitzen die vorliegenden erfindungsgemäßen Substanzen deshalb eine wesentlich verbesserte Selektivität bei Kulturpflanzen.

Die vorliegenden Verbindungen eignen sich aus diesen Gründen sehr gut zur Bekämpfung von unerwünschtem Pflanzenwuchs in landwirtschaftlichen Nutzpflanzungen.

Die Schädigung der Unkrautpflanzen bzw. die Kulturpflanzenverträglichkeit wurde in einem Schlüssel von 0-5 bonitiert.

Dabei bedeutet

0 = ohne Wirkung (Schaden)
1 = 0 - 20 % Wirkung
2 = 20 - 40 % Wirkung
3 = 40 - 60 % Wirkung
4 = 60 - 80 % Wirkung
5 = 80 - 100 % Wirkung

1. Vorauflaufverfahren

Samen bzw. Rhizomstücke mono- und dikotyler Unkräuter wurden in Lehmerde in Plastiktöpfen (Ø 9 cm) ausgelegt und mit Erde abgedeckt. Die als benetzbare Pulver bzw. als Emulsionskonzentrate formulierten erfindungsgemäßen Verbindungen wurden in Form wässriger Suspensionen oder Emulsionen auf die Erdoberfläche appliziert. Die Wasseraufwandmenge pro Topf entsprach dabei umgerechnet 600-800 l/ha. Nach der Behandlung wurden die Versuchstöpfe im Gewächshaus aufgestellt und die Versuchspflanzen unter guten Wachstumsbedingungen (Temperatur: 23 ± 1 °C; rel. Luftfeuchte 60-80 %) kultiviert. Nach ca. 3 Wochen wurde die Pflanzenschädigung optisch bonitiert. Als Vergleich dienten dabei unbehandelte Kontrollen. In Tabelle 3 sind die Vorauflaufergebnisse zusammengefaßt.

Die dargestellten Versuchsergebnisse belegen die ausgezeichnete herbizide Vorauflaufwirkung der neuen, erfindungsgemäßen Verbindungen gegen Ungräser und Unkräuter.

2. Nachauflaufverfahren

Tabelle 2

| Beispiel-Nr. | $R_1$ | X | $R_2$ | $R_3$ | $R_4$ | $R_5$ | $R_6$ | Y | Smp. |
|---|---|---|---|---|---|---|---|---|---|
| 20 | $CH_3$ | O | $CH_3$ | H | H | $OCH_3$ | $OCH_3$ | CH | 166 - 168 |
| 21 | $CH_3$ | O | H | H | H | $CH_3$ | $CH_3$ | CH | 165 |
| 22 | $CH_3$ | O | H | H | H | $OCH_3$ | $CH_3$ | CH | |
| 23 | $CH_3$ | O | H | H | H | $OCH_3$ | $OCH_3$ | CH | |
| 24 | $C_2H_5$ | O | H | H | H | $CH_3$ | $CH_3$ | CH | |
| 25 | $C_2H_5$ | O | H | H | H | $OCH_3$ | $CH_3$ | CH | |
| 26 | $C_2H_5$ | O | H | H | H | $OCH_3$ | $OCH_3$ | CH | |
| 27 | $C_2H_5$ | O | $CH_3$ | H | H | $CH_3$ | $CH_3$ | CH | |
| 28 | $C_2H_5$ | O | $CH_3$ | H | H | $OCH_3$ | $CH_3$ | CH | |
| 29 | $C_2H_5$ | O | $CH_3$ | H | H | $OCH_3$ | $OCH_3$ | CH | |
| 30 | $nC_3H_7$ | O | $CH_3$ | H | H | $CH_3$ | $CH_3$ | CH | Harz |
| 31 | $nC_3H_7$ | O | $CH_3$ | H | $CH_3$ | $CH_3$ | $CH_3$ | CH | |
| 32 | $nC_3H_7$ | O | $CH_3$ | H | H | $OCH_3$ | $CH_3$ | CH | |
| 33 | iso$C_3H_7$ | O | $CH_3$ | H | H | $CH_3$ | $CH_3$ | CH | |
| 34 | iso$C_3H_7$ | O | $CH_3$ | H | H | $OCH_3$ | $CH_3$ | CH | |
| 35 | iso$C_3H_7$ | O | $CH_3$ | H | H | $OCH_3$ | $OCH_3$ | CH | |
| 36 | $CH_2=CHCH_2$ | O | $CH_3$ | H | H | $CH_3$ | $CH_3$ | CH | 126 |

Tabelle 2 (Fortsetzung)

| Bsp.-Nr. | $R_1$ | X | $R_2$ | $R_3$ | $R_4$ | $R_5$ | $R_6$ | Y | Smp. |
|---|---|---|---|---|---|---|---|---|---|
| 37 | $CH_2=CHCH_2$ | O | $CH_3$ | H | H | $OCH_3$ | $CH_3$ | CH | |
| 38 | $CH_2=CH-CH_2$ | O | $CH_3$ | H | H | $OCH_3$ | $OCH_3$ | CH | |
| 39 | $CH_2=CH-CH_2$ | O | $CH_3$ | H | H | Cl | $CH_3$ | CH | |
| 40 | $CH_2=CHCH_2$ | O | $CH_3$ | H | H | $CH_2SCH_3$ | $OCH_3$ | CH | |
| 41 | $CH_2=CHCH_2$ | O | $CH_3$ | H | H | $CH_2OCH_3$ | $CH_3$ | CH | |
| 42 | $CH_3CH_2CH_2CH_2$ | O | $CH_3$ | H | H | $CH_3$ | $CH_3$ | CH | 100 |
| 43 | $CH_3CH_2CH_2CH_2$ | O | $CH_3$ | H | H | $OCH_3$ | $CH_3$ | CH | |
| 44 | $CH_3CH_2CH_2CH_2$ | O | $CH_3$ | H | H | $OCH_3$ | $OCH_3$ | CH | |
| 45 | $CH_3CH_2CH(CH_3)-$ | O | $CH_3$ | H | H | $CH_3$ | $CH_3$ | CH | |
| 46 | $CH_3CH_2CH(CH_3)-$ | O | $CH_3$ | H | H | $OCH_3$ | $CH_3$ | CH | |
| 47 | $CH_3$ | O | $C_2H_5$ | H | H | $CH_3$ | $CH_3$ | CH | |
| 48 | $CH_3$ | O | $C_2H_5$ | H | H | $OCH_3$ | $CH_3$ | CH | |
| 49 | $CH_3$ | O | $C_2H_5$ | H | H | $OCH_3$ | $OCH_3$ | CH | |
| 50 | $CH_3$ | O | $CH_2CH_2CH_3$ | H | H | $CH_3$ | $CH_3$ | CH | |
| 51 | $CH_3$ | O | $CH_2CH_2CH_3$ | H | H | $OCH_3$ | $CH_3$ | CH | |
| 52 | $CH_3$ | O | $CH(CH_3)_2$ | H | H | $OCH_3$ | $CH_3$ | CH | |

Tabelle 2 (Fortsetzung)

| Bsp.-Nr. | $R_1$ | X | $R_2$ | $R_3$ | $R_4$ | $R_5$ | $R_6$ | Y | Smp. |
|---|---|---|---|---|---|---|---|---|---|
| ... | | | | | | | | | |
| 53 | $C_2H_5$ | O | $CH\langle^{CH_3}_{CH_3}$ | H | H | $OCH_3$ | $OCH_3$ | CH | |
| 54 | $CH_3$ | O | H | H | H | $OCH_3$ | $CH_3$ | N | |
| 55 | $CH_3$ | O | $CH_3$ | H | H | $OCH_3$ | $CH_3$ | N | 172 |
| 56 | $CH_3$ | O | $CH_3$ | H | H | $OCH_3$ | $OCH_3$ | N | |
| 57 | $C_2H_5$ | O | $CH_3$ | H | H | $CH_3$ | $OCH_3$ | N | |
| 58 | $C_2H_5$ | O | $CH_3$ | H | H | $OCH_3$ | $OCH_3$ | N | |
| 59 | $C_2H_5$ | O | $CH_3$ | H | H | $CH_2Cl$ | $OCH_3$ | N | |
| 60 | $C_2H_5$ | O | $CH_3$ | H | H | $CH_2OCH_3$ | $OCH_3$ | N | |
| 61 | $C_2H_5$ | O | $CH_3$ | H | H | $CH(OCH_3)_2$ | $OCH_3$ | N | |
| 62 | $C_2H_5$ | O | $CH_3$ | H | H | $CH_2SCH_3$ | $OCH_3$ | N | |
| 63 | $C_2H_5$ | O | $CH_3$ | H | H | $N(CH_3)_2$ | $OCH_3$ | N | |
| 64 | $C_2H_5$ | O | $CH_3$ | H | H | $OCH_2CH_3$ | $OCH_3$ | N | |
| 65 | $CH_2CH_2CH_3$ | O | $CH_3$ | H | H | $OCH_3$ | $CH_3$ | N | |
| 66 | $CH_2CH_2CH_3$ | O | $CH_3$ | H | H | $OCH_3$ | $OCH_3$ | N | |
| 67 | $CH_2=CH-CH_2$ | O | $CH_3$ | H | H | $OCH_3$ | $OCH_3$ | N | |
| 68 | $CH_2=CH-CH_2$ | O | $CH_3$ | H | H | $OCH_3$ | $OCH_3$ | N | |
| 69 | $CH_3CH_2CH_2CH_2$ | O | $CH_3$ | $CH_3$ | $CH_3$ | $OCH_3$ | $CH_3$ | N | |
| 70 | $CH_3CH_2CH_2CH_2$ | O | $C_2H_5$ | H | H | $N(CH_3)_2$ | $OCH_3$ | N | |
| 71 | $CH_3$ | $SO_2$ | H | H | H | $CH_3$ | $CH_3$ | CH | |
| 72 | $CH_3$ | $SO_2$ | $CH_3$ | H | H | $CH_3$ | $CH_3$ | CH | 148 - 150 |
| 73 | $CH_3$ | $SO_2$ | $CH_3$ | H | H | $OCH_3$ | $OCH_3$ | CH | 175 - 177 |
| 74 | $CH_3$ | $SO_2$ | $C_2H_5$ | H | H | $CH_3$ | $CH_3$ | CH | |
| 75 | $CH_3$ | $SO_2$ | $C_2H_5$ | H | H | $OCH_3$ | $CH_3$ | CH | |

Tabelle 2 (Fortsetzung)

| Bsp.-Nr. | $R_1$ | X | $R_2$ | $R_3$ | $R_4$ | $R_5$ | $R_6$ | Y | Smp. |
|---|---|---|---|---|---|---|---|---|---|
| 76 | $CH_3$ | $SO_2$ | $C_2H_5$ | H | H | $OCH_3$ | $OCH_3$ | CH | |
| 77 | $CH_3$ | $SO_2$ | $CH_3CH_2CH_2$ | H | H | $CH_3$ | $CH_3$ | CH | 148-150 |
| 78 | $CH_3$ | $SO_2$ | $CH_3CH_2CH_2$ | H | H | $OCH_3$ | $CH_3$ | CH | 140-142 |
| 79 | $CH_3$ | $SO_2$ | $CH_2{=}CHCH_2$ | H | H | $CH_3$ | $CH_3$ | CH | 137-141 |
| 80 | $CH_3$ | $SO_2$ | $CH_2{=}CHCH_2$ | H | H | $OCH_3$ | $CH_3$ | CH | 158-160 |
| 81 | $CH_3$ | $SO_2$ | $CH_2{=}CHCH_2$ | H | H | Cl | $CH_3$ | CH | |
| 82 | $CH_3$ | $SO_2$ | $CH_2{=}CHCH_2$ | H | H | $CH_2OCH_3$ | $CH_3$ | CH | |
| 83 | $CH_3$ | $SO_2$ | $CH_2{=}CHCH_2$ | H | H | $CH_2OCH_3$ | $OCH_3$ | CH | |
| 84 | $CH_3$ | $SO_2$ | $CH_2{=}CHCH_2$ | H | H | $CH_2SCH_3$ | $OCH_3$ | CH | |
| 85 | $CH_3$ | $SO_2$ | $CH_2{=}CHCH_2$ | H | H | $N(CH_3)_2$ | $CH_3$ | CH | |
| 86 | $CH_3$ | $SO_2$ | 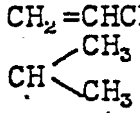 | H | H | $CH_3$ | $CH_3$ | CH | |
| 87 | $CH_3$ | $SO_2$ | 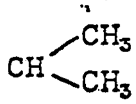 | H | H | $OCH_3$ | $CH_3$ | CH | 160 - 62 |
| 88 | $ClCH_2$ | $SO_2$ | $CH_3$ | H | H | $CH_3$ | $CH_3$ | CH | 144-146 |
| 89 | $ClCH_2$ | $SO_2$ | $CH_3$ | H | H | $OCH_3$ | $CH_3$ | CH | |
| 90 | $ClCH_2$ | $SO_2$ | $CH_3$ | H | H | $OCH_3$ | $OCH_3$ | CH | |
| 91 | $ClCH_2$ | $SO_2$ | $CH_3$ | H | H | $CH(OC_2H_5)_2$ | $OCH_3$ | CH | |
| 92 | $CF_2H$ | $SO_2$ | $CH_3$ | H | H | $CH_3$ | $CH_3$ | CH | Harz |
| 93 | $CF_2H$ | $SO_2$ | $CH_3$ | H | H | $OCH_3$ | $CH_3$ | CH | Harz |
| 94 | $CF_3$ | $SO_2$ | $CH_3$ | H | H | $CH_3$ | $CH_3$ | CH | |
| 95 | $CF_3$ | $SO_2$ | $CH_3$ | H | H | $OCH_3$ | $CH_3$ | CH | |
| 96 | $CHF_2CF_2$ | $SO_2$ | $CH_3$ | H | H | $CH_3$ | $CH_3$ | CH | |
| 97 | $CHF_2CF_2$ | $SO_2$ | $CH_3$ | H | H | $OCH_3$ | $CH_3$ | CH | |

Tabelle 2 (Fortsetzung)

| Bsp.-Nr. | $R_1$ | X | $R_2$ | $R_3$ | $R_4$ | $R_5$ | $R_6$ | Y | Smp. |
|---|---|---|---|---|---|---|---|---|---|
| 98 | $FCH_2$ | $SO_2$ | $CH_3$ | H | H | $CH_3$ | $CH_3$ | CH | |
| 99 | $FCH_2$ | $SO_2$ | $CH_3$ | H | H | $OCH_3$ | $CH_3$ | CH | |
| 100 | $CH_3$ | $SO_2$ | $CH_3$ | H | H | $OCH_3$ | $CH_3$ | N | 72 - 78 |
| 101 | $CH_3$ | $SO_2$ | $CH_3$ | H | H | $OC_2H_5$ | $CH_3$ | N | 77 - 83 |
| 102 | $ClCH_2$ | $SO_2$ | $CH_3$ | H | H | $OCH_3$ | $CH_3$ | N | 125 -128 |
| 103 | $ClCH_2$ | $SO_2$ | $CH_3$ | H | H | $SCH_3$ | $CH_3$ | N | 135 -136 |
| 104 | $CH_3$ | $SO_2$ | $CH_2=CHCH_2$ | H | H | $SCH_3$ | $CH_3$ | N | 116 -118 |
| 105 | $CH_3$ | $SO_2$ | $CH_2=CHCH_2$ | H | H | $OCH_3$ | $CH_3$ | N | 132 -133 |
| 106 | $CH_3$ | $SO_2$ | $CH_3CH_2CH_2$ | H | H | $OCH_3$ | $CH_3$ | N | 98 - 99 |
| 107 | $CH_3$ | $SO_2$ | $CH_3CH_2CH_2$ | H | H | $OCH_3$ | $OCH_3$ | N | 128 -130 |
| 108 | $C_2H_5$ | $SO_2$ | $CH_3$ | H | H | $OCH_3$ | $CH_3$ | N | |
| 109 | $C_3H_7$ | $SO_2$ | $CH_3$ | H | H | $OCH_3$ | $CH_3$ | N | |
| 110 | $CH_2=CH-$ | $SO_2$ | $CH_3$ | H | H | $OCH_3$ | $CH_3$ | N | |
| 111 | $CH\equiv C-CH_2-$ | $SO_2$ | $CH_3$ | H | H | $OCH_3$ | $CH_3$ | N | |
| 112 | $CH\equiv C-CH_2-$ | $SO_2$ | $CH_3$ | H | H | $OCH_3$ | $OCH_3$ | N | |
| 113 | $CF_3$ | $SO_2$ | $CH_3$ | H | H | $OCH_3$ | $CH_3$ | N | |
| 114 | $CHF_2$ | $SO_2$ | $CH_3$ | H | H | $OCH_3$ | $CH_3$ | N | |
| 115 | $CHF_2$ | $SO_2$ | $CH_3$ | H | H | $OCH_3$ | $OCH_3$ | N | |
| 116 | $CH_2F$ | $SO_2$ | $CH_3$ | H | H | $OCH_3$ | $CH_3$ | N | |
| 117 | $CHF_2CF_2$ | $SO_2$ | $CH_3$ | H | H | $OCH_3$ | $CH_3$ | N | |
| 118 | $CF_3CHFCF_2$ | $SO_2$ | $CH_3$ | H | H | $OCH_3$ | $CH_3$ | N | |
| 119 | $CHF_2$ | $SO_2$ | $C_2H_5$ | H | H | $OCH_3$ | $OCH_3$ | N | |
| 120 | $CHF_2$ | $SO_2$ | $CH_3$ | $CH_3$ | H | $OCH_3$ | $CH_3$ | N | |
| 121 | $CHF_2$ | $SO_2$ | $CH_3$ | H | $CH_3$ | $OCH_3$ | $OCH_3$ | N | |
| 122 | $CH_3$ | $SO_2$ | $CH_3CH_2CH_2$ | H | H | $OCH_3$ | $OCH_3$ | CH | 150 - 153 |
| 123 | $CH_3$ | $SO_2$ | $CH(CH_3)_2$ | H | H | $OCH_3$ | $OCH_3$ | CH | 120 - 122 |
| 124 | $CH_3$ | $SO_2$ | $CH(CH_3)_2$ | H | H | Cl | $OCH_3$ | CH | 182 - 184 |

Samen von mono- und dikotylen Unkräutern wurden in Töpfen ausgesät und im Gewächshaus unter guten Wachstumsbedingungen angezogen. 3 Wochen nach der Aussaat wurden die Versuchspflanzen im Dreiblattstadium behandelt. Die als Spritzpulver bzw. als Emulsionskonzentrate formulierten erfindungsgemäßen Präparate wurden in verschiedenen Dosierungen auf die grünen Pflanzenteile gesprüht und nach ca. 3 Wochen Standzeit im Gewächshaus unter optimalen Wachstumsbedingungen (Temperatur : 23 ± 1 °C ; rel. Luftfeuchte 60-80 %) die Wirkung der Präparate optisch im Vergleich zu unbehandelten Kontrollen bonitiert. Die erfindungsgemäßen Mittel wiesen eine gute herbizide Wirksamkeit gegen ein breites Spektrum wirtschaftlich wichtiger annueller und perennierender Unkraüter und Ungräser auf (Tabellen 3, 4).

Abkürzungen :

a.i. = Aktivsubstanz
STM = Stellaria media
AMR = Amaranthus retroflexus
SIA = Sinapis arvensis
LOM = Lolium multiflorum
ECG = Echinochloa crus-galli
AS = Avena sativa

Tabelle 3

Herbizide Wirkung der heterocyclischen Sulfonylharnstoffe im Vorauflauf

| Verbindungs-Nr. | Dosis kg a.i./ha | herbizide Wirkung STM | AMR | SIA | LOM | ECG | AS |
|---|---|---|---|---|---|---|---|
| 17 | 2,5 | 5 | 5 | 5 | 5 | 5 | 4 |
| 18 | 2,5 | 5 | 5 | 5 | 5 | 5 | 4 |
| 19 | 2,5 | 5 | 5 | 5 | 5 | 5 | 4 |
| 20 | 2,5 | 5 | 5 | 5 | 2 | 4 | 1 |
| 21 | 2,5 | 4 | 2 | 1 | 2 | 2 | 0 |
| 36 | 2,5 | 5 | 5 | 5 | 5 | 5 | 1 |
| 42 | 2,5 | 5 | 5 | 5 | 5 | 5 | 1 |
| 55 | 2,5 | 2 | 2 | 0 | 2 | 1 | 0 |
| 72 | 2,5 | 5 | 5 | 5 | 5 | 5 | 5 |
| 77 | 2,5 | 5 | 4 | 5 | 4 | 5 | 3 |
| 78 | 2,5 | 5 | 5 | 5 | 4 | 4 | 3 |
| 79 | 2,5 | 5 | 5 | 5 | 5 | 5 | 5 |
| 80 | 2,5 | 5 | 5 | 5 | 5 | 5 | 4 |
| 88 | 2,5 | 5 | 5 | 5 | 5 | 5 | 3 |
| 100 | 2,5 | 5 | 4 | 5 | 5 | 5 | 2 |
| 101 | 2,5 | 5 | 3 | 3 | 4 | 5 | 2 |
| 102 | 2,5 | 5 | 5 | 5 | 5 | 5 | 3 |
| 103 | 2,5 | 2 | - | - | 2 | - | - |
| 104 | 2,5 | 5 | - | - | 4 | - | - |
| 105 | 2,5 | 5 | 5 | 5 | 5 | 5 | 2 |
| 106 | 2,5 | 5 | 3 | 5 | 4 | 3 | 2 |
| 107 | 2,5 | 5 | - | - | 3 | - | - |
| 73 | 2,5 | 5 | 5 | 5 | 5 | 5 | 5 |
| 87 | 2,5 | 5 | 5 | 5 | 5 | 5 | 5 |
| 122 | 2,5 | 5 | 5 | 5 | 5 | 5 | 5 |

Tabelle 4

Herbizide Wirkung der heterocyclischen Sulfonylharnstoffe im Nachauflauf

| Verbindungs-Nr. | Dosis kg a.i. /ha | herbizide Wirkung SIA | AMR | STM | AS | ECG | LOM |
|---|---|---|---|---|---|---|---|
| 17 | 2,5 | 5 | 5 | 4 | 2 | 4 | 4 |
| 18 | 2,5 | 5 | 5 | 5 | 4 | 5 | 5 |
| 19 | 2,5 | 5 | 5 | 5 | 4 | 5 | 5 |
| 20 | 2,5 | 5 | 5 | 5 | 0 | 2 | 3 |
| 21 | 2,5 | 4 | 0 | 3 | 0 | 0 | 1 |
| 36 | 2,5 | 4 | 4 | 4 | 2 | 4 | 4 |
| 42 | 2,5 | 4 | 5 | 5 | 5 | 3 | 3 |
| 55 | 2,5 | 3 | 0 | 2 | 1 | 0 | 2 |
| 72 | 2,5 | 5 | 5 | 5 | 5 | 5 | 5 |
| 77 | 2,5 | 5 | 5 | 4 | 2 | 4 | 4 |
| 78 | 2,5 | 5 | 5 | 5 | 2 | 4 | 3 |
| 79 | 2,5 | 5 | 5 | 5 | 3 | 5 | 4 |
| 80 | 2,5 | 5 | 5 | 5 | 2 | 5 | 3 |
| 88 | 2,5 | 5 | 5 | 5 | 4 | 5 | 5 |
| 100 | 2,5 | 5 | 5 | 4 | 2 | 5 | 4 |
| 101 | 2,5 | 5 | 5 | 5 | 1 | 5 | 4 |
| 102 | 2,5 | 5 | 5 | 5 | 1 | 4 | 3 |
| 103 | 2,5 | 3 | 4 | 3 | 0 | 2 | 1 |
| 104 | 2,5 | 5 | 5 | 4 | 1 | 3 | 3 |
| 105 | 2,5 | 4 | 5 | 4 | 2 | 5 | 3 |
| 106 | 2,5 | 5 | 5 | 5 | 1 | 5 | 3 |
| 107 | 2,5 | 5 | 5 | 5 | 2 | 4 | 3 |
| 73 | 2,5 | 5 | 5 | 5 | 5 | 5 | 5 |
| 87 | 2,5 | 5 | 4 | 5 | 5 | 5 | 5 |
| 122 | 2,5 | 5 | 5 | 5 | 5 | 5 | 5 |

**Patentansprüche** (für die Vertragstaate : BE, CH, DE, FR, GB, IT, LI, NL, SE)

1. Verbindungen der Formel I,

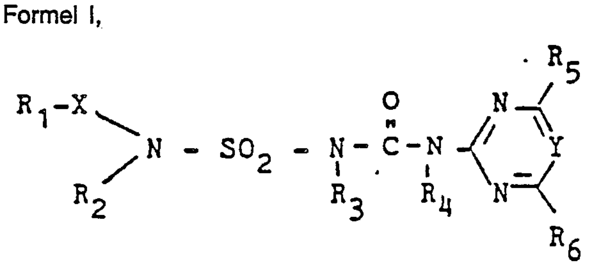

(I)

worin

X O oder $-SO_2-$,

Y CH oder Stickstoff,

$R_1$ einen $(C_1\text{-}C_6)$-Alkyl, $(C_2\text{-}C_6)$-Alkenyl oder einen $(C_2\text{-}C_6)$-Alkinylrest bedeutet, die gegebenenfalls durch Halogen, $(C_1\text{-}C_4)$-Alkoxy oder $(C_1\text{-}C_4)$-Alkoxycarbonyl substituiert sind,

$R_2$ Wasserstoff, $(C_1\text{-}C_6)$-Alkyl, $(C_2\text{-}C_6)$-Alkenyl, $(C_2\text{-}C_6)$-Alkinyl oder Cyclohexyl,

$R_3$, $R_4$ unabhängig voneinander Wasserstoff oder $(C_1\text{-}C_4)$-Alkyl,

$R_5$, $R_6$ unabhängig voneinander Wasserstoff, $(C_1\text{-}C_4)$-Alkyl oder $(C_1\text{-}C_4)$-Alkoxy, die gegebenenfalls ein oder mehrfach durch Halogen, $(C_1\text{-}C_4)$-Alkoxy oder $(C_1\text{-}C_4)$-Alkylthio substituiert sein können, Halogen, $(C_1\text{-}C_4)$-Alkylthio, $(C_1\text{-}C_4)$-Alkylamino oder $(C_1\text{-}C_4)$-Dialkylamino bedeuten,

sowie, falls $R_2$, $R_3$ Wasserstoff bedeuten, deren physiologisch verträgliche Salze mit Basen.

2. Verbindungen der Formel I von Anspruch 1, worin $R_1$, $R_2$ unabhängig voneinander $(C_1\text{-}C_3)$ Alkyl oder Alkenyl mit bis zu 3C-Atomen. $R_3$, $R_4$ Wasserstoff $R_5$, $R_6$ unabhängig voneinander $(C_1\text{-}C_2)$ Alkyl oder $(C_1\text{-}C_2)$ Alkoxy, $X = SO_2$ und $Y = CH$ bedeuten.

3. Verfahren zur Herstellung von Verbindungen der Formel I, dadurch gekennzeichnet, daß man

a) Verbindungen der Formel (III), worin

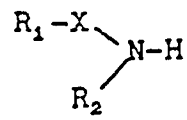

(III)

$R_1$, $R_2$ und X die obengenannten Bedeutungen besitzen, mit Verbindungen der Formel (IV),

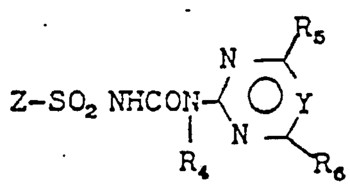

(IV)

worin Z = Fluor, Chlor $(C_1\text{-}C_4)$-Alkoxy- oder Phenoxy, die gegebenenfalls halogeniert sind, insbesondere Chlor bedeutet und $R_4$, $R_5$, $R_6$ und Y den Definitionen für die Verbindungen der Formel (I) entsprechen oder

b) Verbindungen der Formel (II)

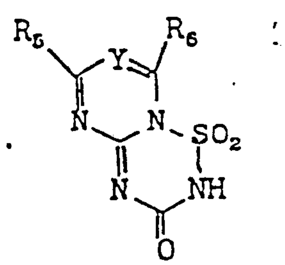

(II)

worin Y, $R_5$ und $R_6$ den Definitionen für Verbindungen der Formel (I) entsprechen, mit Verbindungen der obengenannten Formel (III), oder

c) im Falle $X = SO_2$ Verbindungen der Formel (V),

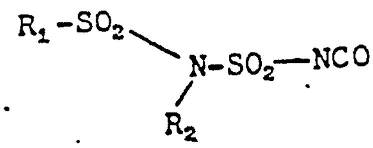

(V)

worin $R_1$ und $R_2$ den für Verbindungen der Formel (I) gegebenen Definitionen entsprechen, mit Verbindungen der Formel (VI), worin $R_4$, $R_5$, $R_6$ und Y die obengenannten Bedeutungen besitzen,

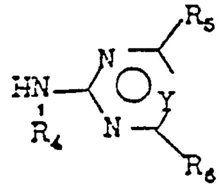

(VI)

umsetzt, und gegebenenfalls die gemäß a) bis c) erhaltenen Verbindungen der Formel (I), worin $R_2$, $R_3$ unabhängig voneinander Wasserstoff bedeuten, in $R_2$ oder $R_3$-Position alkyliert.

4. Herbizide und wachstumsregulierende Mittel, gekennzeichnet durch einen Gehalt an Verbindungen der Formel I.

5. Verwendung von Verbindungen der Formel I zur Bekämpfung von unerwünschtem Pflanzenwuchs und zur Wachstumsregulierung.

6. Verfahren zur Bekämpfung von unerwünschtem Pflanzenwuchs bzw. zur Wachstumsregulierung, dadurch gekennzeichnet, daß man auf die zu behandelnden Pflanzen oder die Anbaufläche eine wirksame Menge einer Verbindung der Formel I aufbringt.

7. Verbindungen der Formel II von Anspruch 3.

**Patentansprüche** (für den Vertragsstaat AT)

1. Verfahren zur Herstellung von Verbindungen der Formel I,

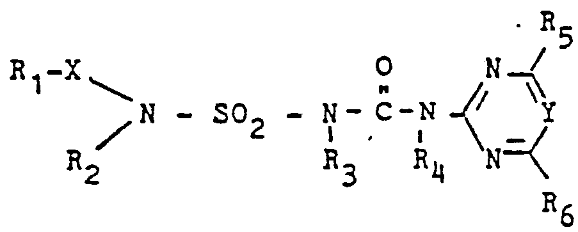

(I)

worin

X O oder $-SO_2-$,

Y CH oder Stickstoff,

$R_1$ einen $(C_1$-$C_6)$-Alkyl, $(C_2$-$C_6)$-Alkenyl oder einen $(C_2$-$C_6)$-Alkinylrest bedeutet, die gegebenenfalls durch Halogen, $(C_1$-$C_4)$-Alkoxy oder $(C_1$-$C_4)$-Alkoxycarbonyl substituiert sind,

$R_2$ Wasserstoff, $(C_1$-$C_6)$-Alkyl, $(C_2$-$C_6)$-Alkenyl, $(C_2$-$C_6)$-Alkinyl oder Cyclohexyl,

$R_3$, $R_4$ unabhängig voneinander Wasserstoff oder $(C_1$-$C_4)$-Alkyl,

$R_5$, $R_6$ unabhängig voneinander Wasserstoff, $(C_1$-$C_4)$-Alkyl oder $(C_1$-$C_4)$-Alkoxy, die gegebenenfalls ein oder mehrfach durch Halogen, $(C_1$-$C_4)$-Alkoxy oder $(C_1$-$C_4)$-Alkylthio substituiert sein können, Halogen, $(C_1$-$C_4)$-Alkylthio, $(C_1$-$C_4)$-Alkylamino oder $(C_1$-$C_4)$-Dialkylamino bedeuten,

sowie, falls $R_2$, $R_3$ Wasserstoff bedeuten, deren physiologisch verträglichen Salzen mit Basen, dadurch gekennzeichnet, daß man

a) Verbindungen der Formel (III), worin

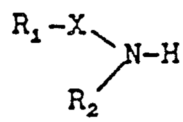

(III)

$R_1$, $R_2$ und X die obengenannten Bedeutungen besitzen, mit Verbindungen der Formel (IV),

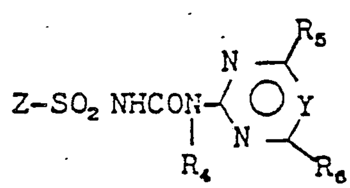

(IV)

worin Z = Fluor, Chlor, $(C_1$-$C_4)$-Alkoxy- oder Phenoxy, die gegebenenfalls halogeniert sind, insbesondere Chlor bedeutet und $R_4$, $R_5$, $R_6$ und Y den Definitionen für die Verbindungen der Formel (I) entsprechen oder

b) Verbindungen der Formel (II)

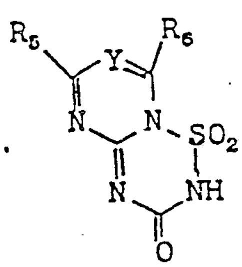

(II)

worin Y, $R_5$ und $R_6$ den Definitionen für Verbindungen der Formel (I) entsprechen, mit Verbindungen der obengenannten Formel (III), oder

c) im Falle X = $SO_2$ Verbindungen der Formel (V),

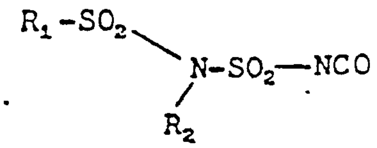

(V)

worin $R_1$ und $R_2$ den für Verbindungen der Formel (I) gegebenen Definitionen entsprechen, mit Verbindungen der Formel (VI), worin $R_4$, $R_5$, $R_6$ und Y die obengenannten Bedeutungen besitzen,

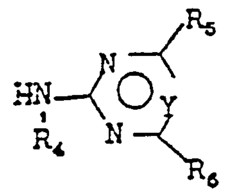

(VI)

umsetzt, und gegebenenfalls die gemäß a) bis c) erhaltenen Verbindungen der Formel (I), worin $R_2$, $R_3$ unabhängig voneinander Wasserstoff bedeuten, in $R_2$ oder $R_3$-Position alkyliert.

2. Verfahren gemäß Anspruch 1, dadurch gekennzeichnet, daß in Formel I von Anspruch 1 $R_1$, $R_2$ unabhängig voneinander ($C_1$-$C_3$) Alkyl oder Alkenyl mit bis zu 3 C-Atomen, $R_3$, $R_4$ Wasserstoff und $R_5$, $R_6$ unabhängig voneinander ($C_1$-$C_2$) Alkyl oder ($C_1$-$C_2$) Alkoxy, X = $SO_2$ und Y = CH bedeuten.

3. Herbizide und wachstumsregulierende Mittel, gekennzeichnet durch einen Gehalt an Verbindungen der Formel I.

4. Verwendung von Verbindungen der Formel I zur Bekämpfung von unerwünschtem Pflanzenwuchs und zur Wachstumsregulierung.

5. Verfahren zur Bekämpfung von unerwünschtem Pflanzenwuchs bzw. zur Wachstumsregulierung, dadurch gekennzeichnet, daß man auf die zu behandelnden Pflanzen oder die Anbaufläche eine wirksame Menge einer Verbindung der Formel I aufbringt.

6. Verfahren zur Herstellung von Verbingungen der Formel II von Anspruch 1, dadurch gekennzeichnet, daß man Chlor- oder Fluorsulfonylisocyanat mit Verbindungen der Formel VI in Gegenwart von Basen umsetzt.

**Claims** (for the Contracting States : BE, CH, DE, FR, GB, IT, LI, NL, SE)

1. A compound of the formula I

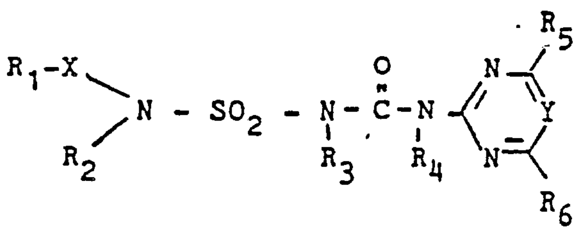

(I)

in which

X denotes O or $—SO_2—$,

Y denotes CH or nitrogen,

$R_1$ denotes a ($C_1$-$C_6$)-alkyl, ($C_2$-$C_6$)-alkenyl or ($C_2$-$C_6$)-alkinyl radical each of which is optionally substituted by halogen, ($C_1$-$C_4$)-alkoxy or ($C_1$-$C_4$)-alkoxycarbonyl,

$R_2$ denotes hydrogen, ($C_1$-$C_6$)-alkyl, ($C_2$-$C_6$)-alkenyl, ($C_2$-$C_6$)-alkinyl or cyclohexyl,

$R_3$ and $R_4$ independently of one another denote hydrogen or ($C_1$-$C_6$)-alkyl, and

$R_5$ and $R_6$ independently of one another denote hydrogen, ($C_1$-$C_4$)-alkyl or ($C_1$-$C_4$)-alkoxy both of which can optionally be monosubstituted or polysubstituted by halogen, ($C_1$-$C_4$)-alkoxy or ($C_1$-$C_4$)-alkylthio, or denote halogen, ($C_1$-$C_4$)-alkylthio, ($C_1$-$C_4$)-alkylamino or ($C_1$-$C_4$)-dialkylamino,

and also, if $R_2$ and $R_3$ denote hydrogen, physiologically acceptable salts thereof with bases.

2. A compound of the formula I as claimed in claim 1, in which $R_1$ and $R_2$ independently of one another denote ($C_1$-$C_3$)-alkyl or alkenyl having up to 3 carbon atoms, $R_3$ and $R_4$ denote hydrogen, $R_5$ and $R_6$ independently of one another denote ($C_1$-$C_2$)-alkyl or ($C_1$-$C_2$-alkoxy, X denotes $SO_2$ and Y denotes CH.

3. A process for the preparation of compounds of the formula I, which comprises
a) reacting compounds of the formula (III)

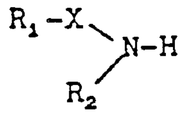

(III)

in which $R_1$, $R_2$ and X have the abovementioned meanings, with compounds of the formula (IV)

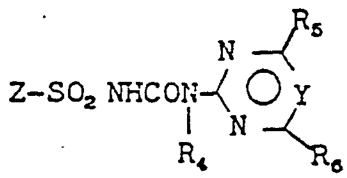

(IV)

in which Z denotes fluorine, chlorine or ($C_1$-$C_4$)-alkoxy or phenoxy which are optionally halogenated, especially chlorine, and $R_4$, $R_5$, $R_6$ and Y correspond to the definitions employed for the compounds of the formula (I), or

b) reacting compounds of the formula (II)

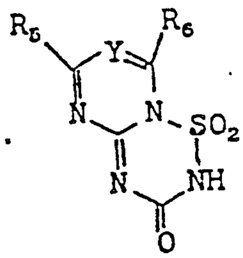

(II)

in which Y, $R_5$ and $R_6$ correspond to the definitions employed for compounds of the formula (I), with compounds of the abovementioned formula (III), or

c) in the event that X = $SO_2$, reacting compounds of the formula (V)

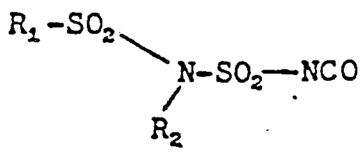

(V)

in which $R_1$ and $R_2$ correspond to the definitions given for compounds of the formula (I), with compounds of the formula (VI)

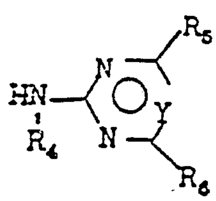

(VI)

in which $R_4$, $R_5$, $R_6$ and Y have the abovementioned meanings, and, if appropriate, alkylating the compounds of the formula (I) obtained in accordance with a) to c) in which $R_2$ and $R_3$ independently of one another denote hydrogen, in the $R_2$-position of $R_3$-position.

4. A herbicidal and growth-regulating agent which contains compounds of the formula I.

5. The use of compounds of the formula I for controlling undesirable plant growth and for regulating growth.

6. A process for controlling undesirable plant growth or for regulating plant growth, which comprises applying an effective amount of a compound of the formula I to the plants to be treated or to the area cultivated.

7. A compound of the formula II as claimed in claim 3.

**Claims** (for the contracting state : AT)

1. A process for the preparation of a compound of the formula I

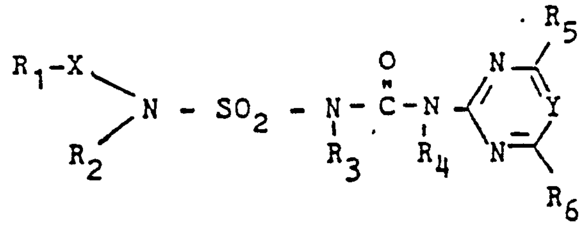

(I)

in which

X denotes O or —$SO_2$—,

Y denotes CH or nitrogen,

$R_1$ denotes a ($C_1$-$C_6$)-alkyl, ($C_2$-$C_6$)-alkenyl or ($C_2$-$C_6$)-alkinyl radical each of which is optionally substituted by halogen, ($C_1$-$C_4$)-alkoxy or ($C_1$-$C_4$)-alkoxycarbonyl,

$R_2$ denotes hydrogen, ($C_1$-$C_6$)-alkyl, ($C_2$-$C_6$)-alkenyl, ($C_2$-$C_6$)-alkinyl or cyclohexyl,

$R_3$ and $R_4$ independently of one another denote hydrogen or ($C_1$-$C_4$)-alkyl, and

$R_5$ and $R_6$ independently of one another denote hydrogen, ($C_1$-$C_4$)-alkyl or ($C_1$-$C_4$)-alkoxy both of which can optionally be monosubstituted or polysubstituted by halogen, ($C_1$-$C_4$)-alkoxy or ($C_1$-$C_4$)-alkylthio, or denote halogen, ($C_1$-$C_4$)-alkylthio, ($C_1$-$C_4$)-alkylamino or ($C_1$-$C_4$)-dialkylamino,

and also, if $R_2$ and $R_3$ denote hydrogen, physiologically acceptable salts thereof with bases which comprises

a) reacting compounds of the formula (III)

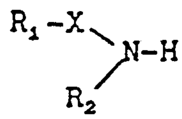

(III)

in which $R_1$, $R_2$ and X have the abovementioned meanings, with compounds of the formula (IV)

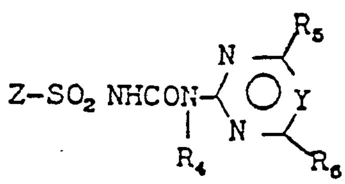

(IV)

in which Z denotes fluorine, chlorine or ($C_1$-$C_4$)-alkoxy or phenoxy which are optionally halogenated, especially chlorine, and $R_4$, $R_5$, $R_6$ and Y correspond to the definitions employed for the compounds of the formula (I), or

b) reacting compounds of the formula (II)

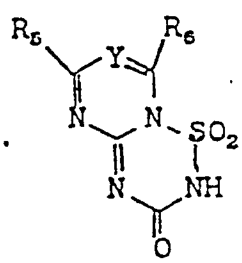

(II)

in which Y, $R_5$ and $R_6$ correspond to the definitions employed for compounds of the formula (I), with compounds of the abovementioned formula (III), or

c) in the event that X = $SO_2$, reacting compounds of the formula (V)

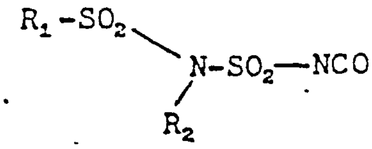

(V)

in which $R_1$ and $R_2$ correspond to the definitions given for compounds of the formula (I), with compounds of the formula (VI)

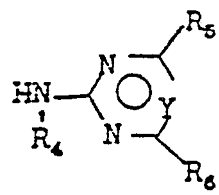

(VI)

in which $R_4$, $R_5$, $R_6$ and Y have the abovementioned meanings, and, if appropriate, alkylating the compounds of the formula (I) obtained in accordance with a) to c) in which $R_2$ and $R_3$ independently of one another denote hydrogen, in the $R_2$-position or $R_3$-position.

2. A process as claimed in claim 1 wherein in formula I as claimed in claim 1, $R_1$ and $R_2$ independently of one another denote ($C_1$-$C_3$)-alkyl or alkenyl having up to 3 carbon atoms, $R_3$ and $R_4$ denote hydrogen, $R_5$ and $R_6$ independently of one another denote ($C_1$-$C_2$)-alkyl or ($C_1$-$C_2$)-alkoxy, X denotes $SO_2$ and Y denotes CH.

3. A herbicidal and growth-regulating agent which contains compounds of the formula I.

4. The use of compounds of the formula I for controlling undesirable plant growth and for regulating growth.

5. A process for controlling undesirable plant growth or for regulating plant growth, which comprises applying an effective amount of a compound of the formula I to the plants to be treated or to the area cultivated.

6. A process for the preparation of compounds of the fomula II as claimed in claim 1, which comprises reacting chlorosulfonyl isocyanate or fluorosulfonyl isocyanate with compounds of the formula VI in the presence of bases.

**Revendications** (pour les Etats contractants : BE, CH, DE, FR, GB, IT, LI, NL, SE)

1. Composés répondant à la formule I :

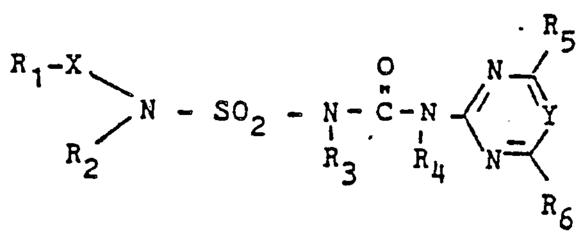

(I)

dans laquelle

X représente —O— ou —$SO_2$—,

Y représente CH ou l'azote,

$R_1$ représente un alkyle en $C_1$-$C_6$, un alcényle en $C_2$-$C_6$ ou un alcynyle en $C_2$-$C_6$, radicaux qui peuvent éventuellement porter un halogène, un alcoxy en $C_1$-$C_4$ ou un ($C_1$-$C_4$) alcoxy-carbonyle,

$R_2$ représente l'hydrogène, un alkyle en $C_1$-$C_6$, un alcényle en $C_2$-$C_6$, un alcynyle en $C_2$-$C_6$ ou un cyclohexyle,

$R_3$ et $R_4$ représentent chacun, indépendamment l'un de l'autre, l'hydrogène ou un alkyle en $C_1$-$C_4$,

$R_5$ et $R_6$ représentent chacun, indépendamment l'un de l'autre, l'hydrogène, un alkyle en $C_1$-$C_4$ ou un alcoxy en $C_1$-$C_4$ [ces radicaux pouvant éventuellement porter un ou plusieurs substituants pris parmi les halogènes, les alcoxy en $C_1$-$C_4$ et les alkylthio en $C_1$-$C_4$], un halogène, un alkylthio en $C_1$-$C_4$, un alkylamino en $C_1$-$C_4$ ou un di-($C_1$-$C_4$) alkyl-amino,

et également, dans le cas où $R_2$ ou $R_3$ représente l'hydrogène, les sels physiologiquement acceptables qu'ils forment avec des bases.

2. Composés de formule I selon la revendication 1 dans lesquels : $R_1$ et $R_2$ représentent chacun, indépendamment l'un de l'autre, un alkyle en $C_1$-$C_3$ ou un alcényle contenant au plus 3 atomes de carbone, $R_3$ et $R_4$ représentent chacun l'hydrogène, $R_5$ et $R_6$ représentent chacun, indépendamment l'un de l'autre, un alkyle en $C_1$ ou $C_2$ ou un alcoxy en $C_1$ ou $C_2$, X représente —$SO_2$— et Y représente CH.

3. Procédé pour préparer des composés de formule I, procédé caractérisé en ce que :

a) on fait réagir des composés répondant à la formule III :

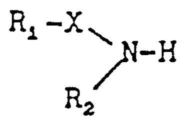

(III)

dans laquelle $R_1$, $R_2$ et X ont les significations indiquées ci-dessus, avec des composés répondant à la formule IV :

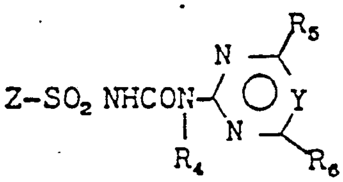

(IV)

dans laquelle Z représente un atome de fluor ou de chlore ou un radical ($C_1$-$C_4$) alcoxy ou phénoxy éventuellement halogéné, mais représente plus particulièrement le chlore, et $R_4$, $R_5$, $R_6$ et Y correspondent aux définitions qui ont été données pour les composés de formule I,

ou

b) on fait réagir des composés répondant à la formule II :

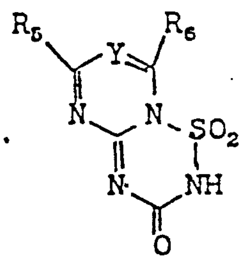

(II)

dans laquelle Y, $R_5$ et $R_6$ correspondent aux définitions qui ont été données pour les composés de formule I, avec des composés répondant à la formule III représentée plus haut,

ou

c) dans le cas où X représente un radical $-SO_2-$ on fait réagir des composés répondant à la formule V :

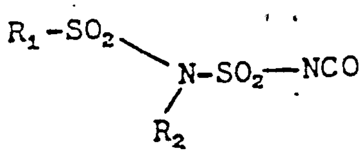

(V)

dans laquelle $R_1$ et $R_2$ correspondent aux définitions qui ont été données pour les composés de formule I, avec des composés répondant à la formule VI :

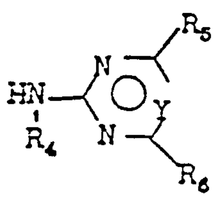

(VI)

dans laquelle $R_4$, $R_5$, $R_6$ et Y ont les significations qui leur ont été précédemment données, et éventuellement on alkyle à la position de $R_2$ ou de $R_3$ les composés de formule I obtenus, dans lesquels $R_2$ et $R_3$ représentent chacun, indépendamment l'un de l'autre, un atome d'hydrogène.

4. Produits herbicides et régulateurs de croissance caractérisés en ce qu'ils contiennent des composés de formule I.

5. Application de composés de formule I à la lutte contre la végétation indésirable et à la régulation de la croissance.

6. Procédé pour combattre la végétation indésirable ou pour régler la croissance, procédé caractérisé en ce qu'on applique sur les plantes à traiter ou sur les surfaces cultivées une quantité efficace d'un composé de formule I.

7. Composés de formule II selon la revendication 3.

**Revendications** (pour l'Etat contractant AT)

1. Procédé pour préparer des composés répondant à la formule I :

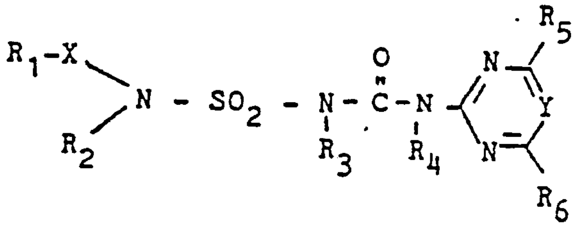

(I)

dans laquelle

X représente —O— ou —$SO_2$—,

Y représente CH ou l'azote,

$R_1$ représente un alkyle en $C_1$-$C_6$, un alcényle en $C_2$-$C_6$ ou un alcynyle en $C_2$-$C_6$, radicaux qui peuvent éventuellement porter un halogène, un alcoxy en $C_1$-$C_4$ ou un ($C_1$-$C_4$) alcoxy-carbonyle,

$R_2$ représente l'hydrogène, un alkyle en $C_1$-$C_6$, un alcényle en $C_2$-$C_6$, un alcynyle en $C_2$-$C_6$ ou un cyclohexyle,

$R_3$ et $R_4$ représentent chacun, indépendamment l'un de l'autre, l'hydrogène ou un alkyle en $C_1$-$C_4$,

$R_5$ et $R_6$ représentent chacun, indépendamment l'un de l'autre, l'hydrogène, un alkyle en $C_1$-$C_4$ ou un alcoxy en $C_1$-$C_4$ [ces radicaux pouvant éventuellement porter un ou plusieurs substituants pris parmi les halogènes, les alcoxy en $C_1$-$C_4$ et les alkylthio en $C_1$-$C_4$], un halogène, un alkylthio en $C_1$-$C_4$, un alkylamino en $C_1$-$C_4$ ou un di-($C_1$-$C_4$) alkyl-amino,

et également, dans le cas où $R_2$ ou $R_3$ représente l'hydrogène, les sels physiologiquement acceptables qu'ils forment avec des bases, procédé caractérisé en ce que :

a) on fait réagir des composés répondant à la formule III :

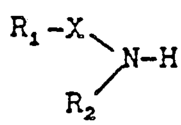

(III)

dans laquelle $R_1$, $R_2$ et X ont les significations indiquées ci-dessus, avec des composés répondant à la formule IV :

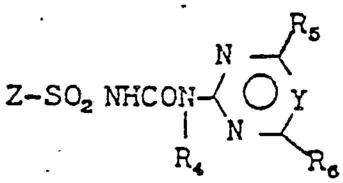

(IV)

dans laquelle Z représente un atome de fluor ou de chlore ou un radical ($C_1$-$C_4$) alcoxy ou phénoxy éventuellement halogéné, mais représente plus particulièrement le chlore, et $R_4$, $R_5$, $R_6$ et Y correspondent aux définitions qui ont été données pour les composés de formule I,

ou

b) on fait réagir des composés répondant à la formule II :

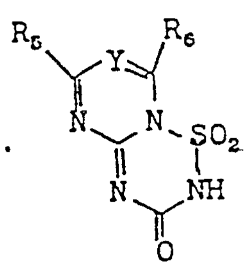

(II)

dans laquelle Y, $R_5$ et $R_6$ correspondent aux définitions qui ont été données pour le composé de formule I, avec des composés répondant à la formule III représentée plus haut,
ou

c) dans le cas où X représente un radical $—SO_2—$ on fait réagir des composés répondant à la formule V :

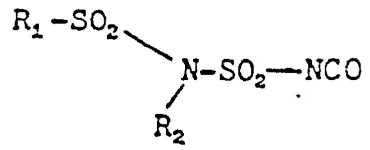

(V)

dans laquelle $R_1$ et $R_2$ correspondent aux définitions qui ont été données pour les composés de formule I, avec des composés répondant à la formule VI :

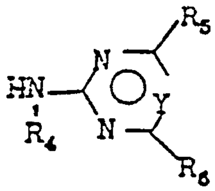

(VI)

dans laquelle $R_4$, $R_5$, $R_6$ et Y ont les significations qui leur ont été précédemment données, et éventuellement on alkyle à la position de $R_2$ ou de $R_3$ les composés de formule I obtenus, dans lesquels $R_2$ et $R_3$ représentent chacun, indépendamment l'un de l'autre, un atome d'hydrogène.

2. Procédé selon la revendication 1 caractérisé en ce que, dans la formule I de la revendication 1 : $R_1$ et $R_2$ représentent chacun, indépendamment l'un de l'autre, un alkyle en $C_1$-$C_3$ ou un alcényle à au plus 3 atomes de carbone, $R_3$ et $R_4$ représentent chacun l'hydrogène, $R_5$ et $R_6$ représentent chacun, indépendamment l'un de l'autre, un alkyle à 1 ou 2 atomes de carbone ou un alcoxy à 1 ou 2 atomes de carbone, X représente $—SO_2—$ et Y représente CH.

3. Produits herbicides et régulateurs de croissance caractérisés en ce qu'ils contiennent des composés de formule I.

4. Application de composés de formule I à la lutte contre la végétation indésirable et à la régulation de la croissance.

5. Procédé pour combattre la végétation indésirable ou pour régler la croissance, procédé caractérisé en ce qu'on applique sur les plantes à traiter ou sur les surfaces cultivées une quantité efficace d'un composé de formule I.

6. Procédé de préparation de composés de formule II selon la revendication 1, procédé caractérisé en ce qu'on fait réagir l'isocyanate de chlorosulfonyle ou de fluorosulfonyle avec des composés de formule VI en présence de bases.